(19) Europäisches Patentamt
European Patent Office
Office européen des brevets

(11) **EP 0 908 445 B1**

(12) # FASCICULE DE BREVET EUROPEEN

(45) Date de publication et mention de la délivrance du brevet:
**12.12.2001 Bulletin 2001/50**

(51) Int Cl.[7]: **C07C 229/18**, C07C 229/26, A61K 7/13

(21) Numéro de dépôt: **98402479.4**

(22) Date de dépôt: **06.10.1998**

(54) **Composition de teinture d'oxydation des fibres kératiniques comprenant un dérivé d'aminoacide en tant que base d'oxydation et nouveaux dérivés d'aminoacides**

Zusammensetzung zum oxidativen Färben von Keratinfasern, die als Oxidationsbase ein Aminosäurederivat enthält, und neue Aminosäurederivate.

Oxidation dyeing composition for keratin fibres containing an amino acid derivative as oxidation base and aminoacid derivatives

(84) Etats contractants désignés:
**DE ES FR GB IT**

(30) Priorité: **08.10.1997 FR 9712555**

(43) Date de publication de la demande:
**14.04.1999 Bulletin 1999/15**

(73) Titulaire: **L'OREAL**
**75008 Paris (FR)**

(72) Inventeurs:
- **Philippe, Michel**
  **91320 Wissous (FR)**
- **Bordier, Thierry**
  **93290 Tremblay en France (FR)**

(74) Mandataire: **Casalonga, Axel**
**BUREAU D.A. CASALONGA - JOSSE**
**Morassistrasse 8**
**80469 München (DE)**

(56) Documents cités:
**EP-A- 0 146 350** **US-A- 3 532 743**
**US-A- 3 931 912**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 908 445 B1

## Description

La présente invention est relative à une composition de teinture des fibres kératiniques, et en particulier des fibres kératiniques humaines, comprenant, à titre de base d'oxydation, au moins un dérivé d'aminoacide substitué sur un groupe amino par un groupe à noyau aromatique, à un procédé de teinture utilisant une telle composition et mettant en oeuvre la révélation par un agent oxydant et aux nouveaux dérivés d'aminoacides dont un groupe amino est substitué par un groupe à noyau aromatique.

Il est connu de teindre les fibres kératiniques, et en particulier les cheveux humains, avec des compositions tinctoriales contenant des précurseurs de colorants d'oxydation comprenant des "bases d'oxydation", en particulier des ortho- ou paraphénylènediamines et des ortho- ou para-aminophénols, ainsi que des coupleurs encore appelés modificateurs de coloration, plus particulièrement des méta-phénylènediamines aromatiques, des méta-aminophénols et des méta-diphénols, qui permettent de modifier et d'enrichir de reflets les colorations "de fond" obtenues par les produits de condensation des bases d'oxydation.

Dans le domaine de la teinture capillaire d'oxydation, il est essentiel de disposer de précurseurs de colorants d'oxydation ayant une puissance tinctoriale élevée pour permettre au formulateur de créer une large palette de nuances. De plus, il est également important que les précurseurs de colorants d'oxydation aient une puissance tinctoriale élevée à pH neutre aux environs de 7 plutôt qu'à pH basique ($pH \geq 9$) afin de respecter l'intégralité de la fibre kératinique et de produire une moindre irritation du cuir chevelu.

C'est à la suite de nombreuses recherches que la demanderesse vient de découvrir qu'il est possible d'obtenir sur les fibres kératiniques des nuances naturelles intenses, en particulier à un pH voisin de la neutralité, en utilisant des dérivés d'aminoacides substitués sur un groupe amino par un groupe à noyau aromatique, à titre de bases d'oxydation.

Le brevet US-A-3 532 743 décrit de tels dérivés d'amino-acides substitués sur le groupe amino par un noyau aromatique comportant un groupe nitro, utilisés à titre de colorants directs dans des compositions de teinture directe ne nécessitant pas d'oxydant pour le développement de la coloration.

Le brevet US-A-3 931 912 décrit des compositions de teinture d'oxydation comprenant la N-(p-hydroxyphényl) glycine comme agent de contrôle ou limitation de production d'oxygène à partir de solutions de peroxyde.

La présente invention a ainsi pour objet une composition de teinture d'oxydation pour fibres kératiniques, en particulier pour fibres kératiniques humaines telles que les cheveux, comprenant dans un milieu approprié pour la teinture, à titre de base d'oxydation, au moins un dérivé d'aminoacide substitué sur un radical amino par un groupe à noyau aromatique ayant pour formule :

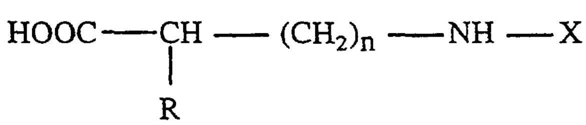

(I)

dans laquelle :

- n est un nombre entier compris entre 0 et 11 inclusivement ;
- R représente un atome d'hydrogène, un radical amino, alkyle en $C_1$-$C_6$ linéaire ou ramifié, saturé ou insaturé, ou un radical choisi parmi les groupements suivants :

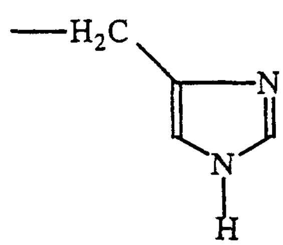

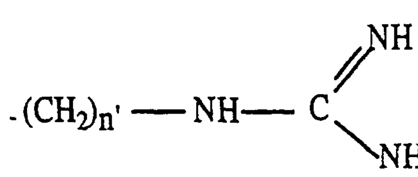

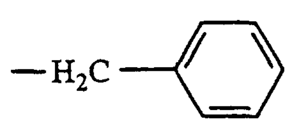

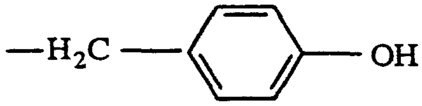

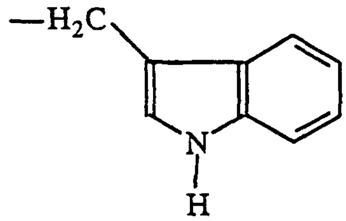

$-(CH_2)_{n'}-COOH$

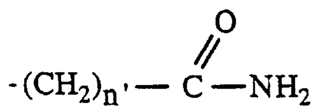

$-(CH_2)_{n'}-SH$

$-(CH_2)_{n'}-SR'$ $-CH_2OH$ $-CHOH-R'$

$-(CH_2)_{n'}-NH-X$ et $-(CH_2)_{n'}-NH_2$

- R' représente 'un radical alkyle en $C_1$-$C_6$, linéaire ou ramifié, saturé ou insaturé ;
- n' est un nombre entier compris entre 1 et 6 inclusivement ;
- X représente un groupe aromatique à un ou deux cycles, le ou les cycles pouvant contenir un ou plusieurs hétéroatomes choisis parmi S, N ou O ; le ou les cycles étant substitués par au moins un groupement -NHR" ou par un radical -OH se trouvant en position para ou ortho sur le groupe aromatique X par rapport au groupement -NH, R" désignant un atome d'hydrogène, un radical alkyle en $C_1$-$C_6$, linéaire ou ramifié, saturé ou insaturé, éventuellement hydroxylé, sans comporter de groupements nitro, à l'exclusion du composé dans lequel n=0, R=H et X est un radical 4-hydroxyphényle,

ou l'un de ses sels d'addition avec un acide.

Les composés (I) peuvent être des isomères optiquement actifs ou des mélanges de tels isomères.

Les nouvelles teintures ainsi obtenues permettent d'obtenir des colorations naturelles soutenues à pH neutre aux environs de 7 et non toxiques.

Ces nouvelles teintures présentent en outre une bonne résistance à la lumière, aux lavages, aux intempéries, à la transpiration et aux différents traitements que peuvent subir les cheveux.

Un autre objet de l'invention est la composition de teinture prête à l'emploi, contenant au moins un dérivé d'aminoacide tel que défini ci-dessus à titre de base d'oxydation et un agent oxydant.

L'invention vise également un procédé de teinture des fibres kératiniques, et en particulier des fibres kératiniques humaines telles que les cheveux, consistant à appliquer sur ces fibres au moins une composition (A) contenant, dans un milieu approprié pour la teinture, à titre de base d'oxydation, au moins un dérivé d'aminoacide de formule (I) telle que définie ci-dessus et à révéler la couleur en milieu alcalin, neutre ou acide à l'aide d'un agent oxydant qui est ajouté juste au moment de l'emploi à la composition (A) ou qui est présent dans une composition (B) appliquée simultanément ou séquentiellement de façon séparée.

L'invention a également pour objet des dispositifs de teinture ou "kits" à plusieurs compartiments, dont le premier compartiment contient une composition (A) comprenant au moins un dérivé d'aminoacide tel que défini ci-dessus à titre de base d'oxydation, et le deuxième compartiment contient une composition (B) comprenant un agent oxydant dans un milieu approprié pour la teinture.

Les dérivés d'aminoacides comportant un groupe amino substitué par un noyau aromatique de formule (I) ci-dessus n'ont jamais été utilisés comme bases d'oxydation en teinture capillaire.

Un dérivé de glycine, à savoir la N-(4-aminophényl) glycine, est connu en thermographie comme réactif de transfert dans un procédé de duplication décrit dans le brevet français FR-A-1.506.081 et en photographie selon le brevet GB-A-1.227.395 ou la demande de brevet JP-A-02068546.

La présente invention a donc également pour objet de nouveaux dérivés d'aminoacides substitués sur un radical amino par un groupe à noyau aromatique de formule :

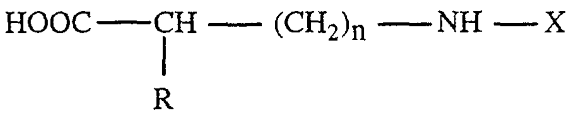 (I')

dans laquelle :

- n est un nombre entier compris entre 0 et 11 inclusivement ;
- R représente un atome d'hydrogène, un radical amino, alkyle en $C_1$-$C_6$ linéaire ou ramifié, saturé ou insaturé, ou un radical choisi parmi les groupements suivants :

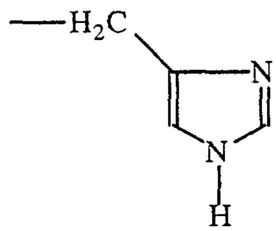

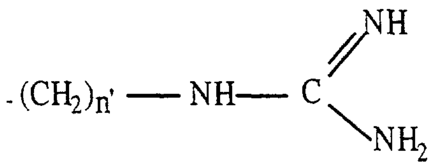

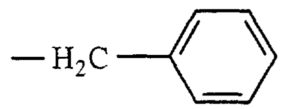

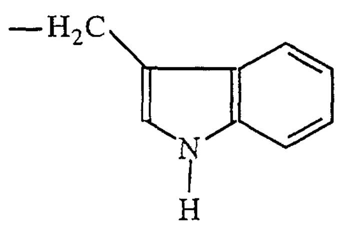

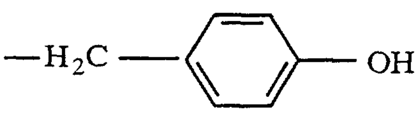

$-(CH_2)_{n'}$—COOH 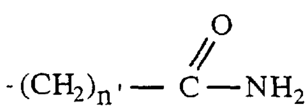 $-(CH_2)_{n'}$—SH

$-(CH_2)_{n'}$—SR' $-CH_2OH$ -CHOH—R'

$-(CH_2)_{n'}$—NH—X et $-(CH_2)_{n'}$—$NH_2$

- R' représente un radical alkyle en $C_1$-$C_6$, linéaire ou ramifié, saturé ou insaturé ;
- n' est un nombre entier compris entre 1 et 6 inclusivement ;
- X représente un groupe aromatique à un ou deux cycles, le ou les cycles pouvant contenir un ou plusieurs hétéroatomes choisis parmi S, N ou O ; le ou les cycles étant substitués par au moins un groupement -NHR" ou par un radical -OH se trouvant en position para ou ortho sur le groupe aromatique X par rapport au groupement -NH, R" désignant un atome d'hydrogène, un radical alkyle en $C_1$-$C_6$, linéaire ou ramifié, saturé ou insaturé, éventuellement hydroxylé, sans comporter de groupements nitro,

à l'exclusion des composés dans lesquels n = 0, R= H et X est un radical 4-aminophényle, 4-hydroxyphényle, 3-hydroxy-2-pyridyle ou 4-hydroxy-3-pyridyle, du composé dans lequel n=0, R= $-CH_3$ et X est un radical 3-hydroxy-2-pyridyle et du composé dans lequel n=0, R=$-CH_2OH$ et X est un radical 3,4-dihydroxy-6-méthylphényle,

ou l'un de leurs sels d'addition avec un acide.

Ces nouveaux dérivés d'aminoacides (I') peuvent être des isomères optiquement actifs ou des mélanges de

tels isomères.

Dans la formule (I) ou (I'), X représente de préférence un cycle phényle ou pyridyle substitué par un groupement amino $-NH_2$.

Les composés (I') nouveaux préférés sont :

- la Nε-(4-aminophényl)-L-lysine ;
- la Nε-(4-amino-2-pyridyl)-L-lysine ;
- la Nα-(4-aminophényl)-L-lysine ;
- la Nα,Nε-di-(4-aminophényl)-L-lysine ;
- la N-(4-aminophényl)-L-sérine et
- l'acide 6-N-(4-aminophényl)amino-caproïque et
- l'α-N-(4-aminophényl)amino -ε-caprolactame.

ainsi que leurs sels d'addition avec un acide.

Les sels d'addition avec un acide sont choisis parmi les chlorhydrates, les sulfates, les bromhydrates et les tartrates.

Les composés (I) préférés pour être utilisés en teinture d'oxydation selon l'invention sont : la Nε-(4-aminophényl)-L-lysine, la Nε-(4-amino-2-pyridyl)-L-lysine ; la N-(4-aminophényl)-glycine, la Nα-(4-aminophényl)-L-lysine, la Nα,Nε-di-(4-aminophényl)-L-lysine, la N-(4-aminophényl)-L-sérine, l'acide 6-N-(4-aminophényl)aminocaproïque et l'α-N-(4-aminophényl) amino -ε-caprolactame, ainsi que leurs sels d'addition avec un acide.

Les composés de formule (I) ou (I') sont préparés suivant les modes opératoires suivants selon qu'il s'agit de dérivés de la lysine, de la glycine ou d'autres dérivés tels que les ω-aminoacides et les dérivés lactames.

**DERIVES DE LA LYSINE**

**Préparation des dérivés nitrés aromatiques en Nε de la lysine.**

Mode opératoire général:

Dans un tricol de 500 ml muni d'un thermomètre et d'une ampoule à introduction de 50 ml, 10 g (54,75 mmoles) de L-lysine monochlorhydrate sont dissous dans 44 ml (2 éq) d'une solution aqueuse de soude à 10 % et 120 ml d'eau. Une solution de 6,8 g (0,5 éq) de sulfate de cuivre pentahydrate dans 40 ml d'eau est introduite dans le milieu réactionnel, suivie de 4,6 g (1 éq) d'hydrogénocarbonate de sodium et 40 ml d'éthanol. Une solution contenant 1 équivalent de 4-fluoro-nitrobenzène ou de 2-chloro-5-nitropyridine dans 40 ml d'éthanol est préparée puis coulée goutte à goutte sur le mélange homogène. Après 6 heures de chauffage au reflux, le milieu réactionnel est refroidi puis filtré. Le précipité jaune-vert correspondant au produit final sous forme de complexe de cuivre, est lavé à l'eau et à l'acétone, puis séché à l'étuve sous vide. Pour éliminer le cuivre, x moles de complexe sont traités par 2 éq de thioacétamide au reflux de l'eau. Après avoir éliminé le sulfure de cuivre par filtration sur papier, le filtrat est refroidi pour obtenir par cristallisation le produit final attendu.

**Exemple 1:** Nε-(4-nitrophényl)-L-lysine

Selon le protocole opératoire décrit précédemment, on obtient à partir du 4-fluoro-nitrobenzène, 4,1 g d'un produit jaune, soit un rendement de 46%.

Analyses :

- F: 254°-258° C (banc Kofler)

- Analyse élémentaire ($C_{12}H_{17}N_3O_4$, PM= 267,287)

| | C | H | N | O |
|---|---|---|---|---|
| % calc. | 53,92 | 6,41 | 15,72 | 23,94 |
| % trouv. | 53,64 | 6,36 | 15,85 | 24,16 |

**Exemple 2:** Nε-(4-nitro-2-pyridyl)-L-lysine

Selon le protocole opératoire décrit précédemment, on obtient à partir de la 2-chloro-5-nitropyridine, 10,7 g d'un produit jaune, soit un rendement de 73%.

Analyses:

- F: > 260° C (banc Kofler)
- Analyse élémentaire ($C_{11}H_{16}N_4O_4$, PM= 268,275)

| | C | H | N | O |
|---|---|---|---|---|
| % calc.<br>% trouv. | 49,25<br>48,96 | 6,01<br>6,09 | 20,88<br>20,95 | 23,86<br>24,10 |

**Réduction des dérivés nitrés en para de la Nε-lysine.**

**Exemple 3 :**

Synthèse de la Nε-(4-aminophényl)-L-lysine, trichlorhydrate

Dans un réacteur en verre (appareil de PAAR) de 250ml, 2 g (7,48 mmoles) de Nε-(4-nitrophényl)-L-lysine sont dissous dans 30 ml d'éthanol, 9 ml d'eau et 5,2 ml (3,5 éq) d'acide chlorhydrique 5N. Puis 2 g de palladium sur charbon humide à 50 % et actif à 10% sont introduits. Le mélange est hydrogéné sous une pression d'environ 2,76 x $10^5$ Pa pendant 1 heure et à la température de 10°C. Après filtration sur célite, le filtrat est évaporé. Le résidu huileux violacé est repris par de l'éthanol et précipité dans un grand volume d'acétone sous agitation. On obtient après filtration, 2,6 g d'une poudre cristalline blanche soit un rendement final de 99%.

Analyses:

- F: 146-150° C (banc Kofler)
- Analyse élémentaire ($C_{12}H_{19}N_3O_2$,3HCl; PM= 346,687)

| | C | H | N | O | Cl |
|---|---|---|---|---|---|
| % calc.<br>% trouv. | 41,57<br>41,04 | 6,40<br>6,55 | 12,12<br>11,78 | 9,23<br>10,57 | 30,68<br>30,28 |

**Exemple 4 :**

Synthèse de la Nε-(4-amino-2-pyridyl)-L-lysine, trichlorhydrate

Dans un tricol de 100 ml muni d'un thermomètre et d'un réfrigérant, 4 g (14,9 mmoles) de Nε-(4-nitro-2-pyridyl)-L-lysine sont mis en suspension dans 80 ml d'éthanol et 8 ml d'eau. Puis sont introduits 6 g de palladium sur charbon (actif à 10% et humide à 50%) suivis de 40 ml de cyclohexène. Le mélange est porté au reflux pendant 2 heures puis est refroidi avant d'être filtré sur célite. Après avoir versé 60 ml (4 éq) d'acide chlorhydrique 1 N sur le filtrat, celui-ci est évaporé à sec, repris par de l'éthanol et précipité dans de l'acétone. On obtient après filtration, 3,4 g de produit soit un rendement final de 66 %.

Analyses:

- F: 180-185° C (banc Kofler)
- Analyse élémentaire ($C_{11}H_{18}N_4O_2$,3HCl; PM= 347,675)

| | C | H | N | O | Cl |
|---|---|---|---|---|---|
| % calc. | 38,00 | 6,09 | 16,11 | 9,2 | 30,59 |
| % trouv. | 38,03 | 6,05 | 15,97 | 9,55 | 30,43 |

**Préparation des dérivés nitrés aromatiques en Nα de la lysine.**

**Exemple 5 :**

Synthèse de la Nα-(4-nitrophényl)-Nε-(benzyloxycarbonyl)-L-lysine

Dans un tricol de 250 ml muni d'un réfrigérant et d'un thermomètre, 5 g (17,83 mmoles) de Nε-benzyloxycarbonyl-L-lysine sont dissous dans 130 ml d'eau en présence de 0,71 g (1 éq) de soude et 2,25 g (1,5 éq) de bicarbonate de soude. Une solution de 2,85 ml (1,5 éq) de 4-fluoro-nitrobenzène dans 70 ml d'éthanol est versée dans le mélange réactionnel lequel est ensuite porté au reflux pendant 48 heures. Le mélange est neutralisé par de l'acide chlorhydrique 1 N, évaporé puis extrait par de l'acétate d'éthyle. La phase aqueuse est acidifiée à pH~3 et à nouveau extraite par de l'acétate d'éthyle. Les phases organiques sont réunies, lavées à l'eau et séchées sur sulfate de sodium. Après évaporation, on obtient une huile jaune-orangée que l'on purifie par flash chromatographie (éluant: $CH_2Cl_2$ 95 / $CH_3OH$ 5) pour donner 1,6 g d'un produit cristallin jaune soit un rendement final de 22 %.

Analyses:

- F: 136°-140° C (banc Kofler)
- HPCCM (silice MERCK): éluant acétate d'éthyle 80/ méthanol 20
  Rf = 0,30 (révélation UV)
- Les spectres RMN sont enregistrés sur un BRUKER AMX 500 MHz et sont conformes à la structure attendue.

**Réduction des dérivés nitrés en para de la Nα-lysine**

**Exemple 6 :**

Synthèse de la Nα-(4-aminophényl)-L-lysine, trichlorhydrate

Dans un réacteur en verre (appareil de PAAR) de 250ml, 0,40 g (1 mmole) de Nα-(4-nitrophényl)-Nε-(benzyloxycarbonyl)-L-lysine est dissous dans 20 ml d'éthanol, 5 ml d'eau et 0,5 ml (3,5 éq) d'acide chlorhydrique 5N. Puis 0,5 g de palladium sur charbon (humide à 50 % et actif à 10%) est introduit. Le mélange est hydrogéné sous une pression d'environ 2,76 x $10^5$ Pa pendant 45 minutes et à la température de 12°C. Après filtration sur célite, le filtrat est évaporé. Le résidu huileux violacé est repris par de l'éthanol et précipité dans un grand volume d'acétone sous agitation. On obtient après filtration, 0,3 g d'une poudre cristalline blanche soit un rendement final de 93%.

- Analyse élémentaire ($C_{12}H_{19}N_3O_2$,3HCl, $2H_2O$; PM= 382,7)

| | C | H | N | O | Cl |
|---|---|---|---|---|---|
| % calc. | 37,62 | 6,27 | 10,97 | 16,72 | 27,82 |
| % trouv. | 37,00 | 6,63 | 10,35 | 15,28 | 27,44 |

**Préparation des dérivés nitrés aromatiques en Nα et Nε de la lysine.**

**Exemple 7 :**

Synthèse de la Nα,Nε-di-(4-nitrophényl)-L-lysine

Dans un tricol de 250 ml muni d'un réfrigérant et d'un thermomètre, 5 g (34,2 mmoles) de L -lysine sont dissous dans 100 ml d'eau en présence de 1,4 g (1 éq) de soude et 8,6 g (3 éq) de bicarbonate de soude. Une solution de 10,8 ml (3 éq) de 4-fluoronitrobenzène dans 60 ml d'éthanol est versée au mélange lequel est porté au reflux (85°-90°C) pendant 5 jours. Le mélange refroidi est extrait avec de l'éther éthylique. La phase aqueuse est acidifiée jusqu'à pH~3 par de l'acide chlorhydrique 5 N. Un précipité gommeux se forme lequel est extrait par du dichlorométhane en présence d'un peu de méthanol. La phase organique est lavée à l'eau jusqu'à neutralité, séchée sur sulfate de sodium puis évaporée pour donner une huile orangée qui cristallise en présence d'éther éthylique. On obtient 11,7 g d'un produit cristallin jaune soit un rendement de 88 %.

Analyses:

- F : 72°-76° C (banc Kofler)

Analyse élémentaire ($C_{18}H_{20}N_4O_6$, PM= 388,38)

| | C | H | N | O |
|---|---|---|---|---|
| % calc. | 55,67 | 5,19 | 14,43 | 24,72 |
| % trouv. | 55,90 | 5,13 | 14,79 | 24,96 |

**Réduction des dérivés nitrés en para de la Nα et Nε lysine**

**Exemple 8 :**

Synthèse de la Nα,Nε-di-(4-aminophényl)-L-lysine, tétrachlorhydrate

Dans un réacteur en verre (appareil de PAAR) de 250ml, 1,5 g (3,86 mmoles) de Nα,Nε-di(4-nitrophényl)-L-lysine sont dissous dans 35 ml d'éthanol, 7 ml d'eau et 3,5 ml (3,5 éq) d'acide chlorhydrique 5N. Puis 3 g de palladium sur charbon (humide à 50 % et actif à 10%) sont introduits. Le mélange est hydrogéné sous une pression d'environ 2,76 x $10^5$ Pa pendant 1 heure et à la température de 10°C. Après filtration sur célite, le filtrat est évaporé. Le résidu huileux violacé est repris par de l'éthanol et précipité dans un grand volume d'acétone sous agitation. On obtient après filtration, 0,8 g d'une poudre cristalline blanche soit un rendement final de 44%.

Analyses:

- HPCCM (silice MERCK): éluant $NH_4OH$ 6/ $CH_3OH$ 47/ $CH_2Cl_2$ 47
  Rf = 0,75 (révélation UV et ninhydrine)
- Les spectres RMN sont enregistrés sur un BRUKER AMX 400 MHz et sont conformes à la structure attendue.

**DERIVES DE LA GLYCINE**

**Préparation des dérivés nitrés aromatiques de la glycine.**

**Exemple 9 :**

Synthèse de la N-(4-nitrophényl)-glycine

Dans un tricol de 250 ml muni d'un réfrigérant et d'un thermomètre, 7,5 g (0,1 mole) de glycine sont dissous dans 100 ml d'eau en présence de 4 g (1 éq) de soude et 8,4 g (1 éq) de bicarbonate de soude. Une solution de 14,1 g (1 éq) de 4-fluoro-nitrobenzène dans 60 ml d'éthanol est versée au mélange lequel est porté au reflux (85°-90°C) pendant 5 heures. Le mélange est refroidi puis extrait 3 fois par de l'acétate d'éthyle. La phase aqueuse est acidifiée jusqu'à pH~3 par de l'acide chlorhydrique 5N. Un précipité jaune se forme; on laisse une nuit au réfrigérateur .On essore et rince par de l'acétone . On obtient 10,8 g d'un produit cristallin jaune .Les filtrats sont concentrés au quart de leur volume initial .Un précipité jaune apparaît et est isolé par filtration .On obtient 7,3 g soit un poids total de 18,1 g (rendement de 83% ).

Analyses:

- HPCCM (silice MERCK) : éluant $NH_4OH$ 1/ $CH_3OH$ 4/ $CH_2Cl_2$ 15
  Rf = 0,4 (révélateur $UV_{254nm}$ et ninhydrine).

**Réduction des dérivés nitrés aromatiques de la glycine**

**Exemple 10 :**

Synthèse de la N-(4-aminophényl)-glycine, 2HCl

Dans un réacteur en verre (appareil de PAAR) de 250ml, 1 g (5 mmoles) de (4-nitrophényl)-glycine est dissous dans 20 ml d'éthanol, 5 ml d'eau et 2,5 ml (2,5 éq) d'acide chlorhydrique 5N. Puis 1 g de palladium sur charbon (humide à 50 % et actif à 10%) sont introduits. Le mélange est hydrogéné sous une pression d'environ 2,76 x $10^5$ Pa pendant 1 heure 30 minutes à la température de 20°C. Après filtration sur célite, le filtrat est évaporé à sec. On obtient un solide blanc que l'on reprend dans 50 ml d'acétone, filtre et sèche sous vide à 40°C. On obtient 0,95 g d'une poudre blanche.

- Analyse élémentaire ($C_8H_{10}N_2O_2$, 2HCl, PM= 239,103 )

| | C | H | N | O | Cl |
|---|---|---|---|---|---|
| % calc. | 40,19 | 5,06 | 11,72 | 13,38 | 29,66 |
| % trouv. | 39,85 | 5,52 | 11,19 | 14,97 | 28,71 |

**AUTRES DERIVES:**

**$\bar{\omega}$ AMINO-ACIDES**

**Exemple 11 :**

Synthèse de l'acide 6-N-(4-nitrophényl)amino-caproïque

Dans un tricol de 250 ml muni d'un réfrigérant et d'un thermomètre, 5 g (38,11 mmoles) d'acide 6-aminocaproïque sont dissous dans 50 ml d'eau en présence de 1,5 g (1 éq) de soude et 4,2 g (1,3 éq) de bicarbonate de soude. Une solution de 4,9 ml (1,2 éq) de 4-fluoro-nitrobenzène dans 50 ml d'éthanol est versée au mélange lequel est ensuite porté au reflux (85°-90°C) pendant 48 heures. Le mélange est refroidi puis extrait 3 fois par du dichlorométhane. La phase aqueuse est acidifiée jusqu'à pH~3 par de l'acide chlorhydrique 5N. Un précipité jaune se forme ; on laisse une nuit au réfrigérateur. Le précipité est essoré, lavé à l'eau et à l'acétone. Après séchage, on obtient 9,3 g d'un produit

jaune soit un rendement final de 97%.

Analyses :

- F: 160°- 162° C (banc Kofler)
- Analyse élémentaire ($C_{12}H_{16}N_2O_4$, PM= 252,272)

| | C | H | N | O |
|---|---|---|---|---|
| % calc.<br>% trouv. | 57,13<br>56,86 | 6,39<br>6,38 | 11,1<br>10,99 | 25,37<br>25,51 |

**Exemple 12 :**

Synthèse de l'acide 6-N-(4-aminophényl)amino-caproïque, dichlorhydrate

Dans un tricol de 150 ml muni d'un thermomètre et d'un réfrigérant 2 g (7,9 mmoles) d'acide 6-N-(4-nitrophényl) amino-caproïque sont partiellement dissous dans 40 ml d'éthanol et 2 ml d'eau. Puis sont introduits 2 g de palladium sur charbon (actif à 10% et humide à 50%) suivis de 20 ml de cyclohexène. Le mélange est porté au reflux pendant 2 heures puis est refroidi avant d'être filtré sur célite. Après avoir versé 4 ml (2,5 éq) d'acide chlorhydrique 5N sur le filtrat, celui-ci est évaporé à sec, repris par de l'éthanol et précipité dans de l'acétone. On obtient après filtration, 1,9 g d'un produit blanc soit un rendement final de 81%.

- Analyse élémentaire ($C_{12}H_{18}N_2O_2$, 2HCl; PM= 295,211)

| | C | H | N | O | Cl |
|---|---|---|---|---|---|
| % calc.<br>% trouv. | 48,82<br>48,43 | 6,83<br>6,79 | 9,49<br>9,43 | 10,84<br>11,55 | 24,02<br>23,17 |

**DERIVES LACTAMES**

**Exemple 13 :**

Synthèse de l'α-N-(4-nitrophényl)amino-ε-caprolactame

Dans un tricol de 100 ml muni d'un réfrigérant et d'un thermomètre, 5 g (39 mmoles) d'α-amino-ε-caprolactame sont dissous dans 50 ml de diméthylformamide et 5,5 ml (1 éq) de triéthylamine. Puis 4,1 ml (1,5 éq) de 4-fluoro-nitrobenzène sont versés au mélange réactionnel lequel est ensuite porté à la température de 70°C pendant 48 heures. Le mélange est refroidi puis est précipité dans l'eau. Un précipité jaune orangé est isolé par filtration puis est purifié sur une colonne de silice (éluant : dichlorométhane 95-méthanol 5). On obtient 2,7 g d'un produit jaune soit un rendement final de 28%.

Analyses :

- F: 216°-218°C (banc Kofler)

Analyse élémentaire ($C_{12}H_{15}N_3O_3$, PM= 249,272)

| | C | H | N | O |
|---|---|---|---|---|
| % calc.<br>% trouv. | 52,82<br>56,97 | 6,07<br>6,15 | 16,86<br>16,54 | 19,26<br>19,07 |

**Exemple 14 :**

Synthèse de l'α-N-(4-aminophényl)amino -ε-caprolactame, dichlorhydrate

Dans un tricol de 150 ml muni d'un thermomètre et d'un réfrigérant, 2 g (8 mmoles) d'α-N-(4-nitrophényl) amino -ε-caprolactame sont partiellement dissous dans 40 ml d'éthanol à 95°. Puis sont introduits 2 g de palladium sur charbon (actif à 10 % et humide à 50 %) suivi de 20 ml de cyclohexène. Le mélange est porté au reflux pendant 3 heures puis est refroidi avant d'être filtré sur célite. Après avoir versé environ 6 ml (~3,5 éq) d'acide chlorhydrique 5N sur le filtrat, celui-ci est évaporé à sec, repris par de l'éthanol et précipité dans de l'acétone. On obtient après filtration, 1,9 g d'un produit blanc soit un rendement final de 81 %.

- Analyse élémentaire ($C_{12}H_{17}N_3O$, 2HCl,$H_2O$;PM=310,2)

| | C | H | N | O | Cl |
|---|---|---|---|---|---|
| % calc. | 46,42 | 6,77 | 13,54 | 10,31 | 22,88 |
| % trouv. | 47,25 | 6,82 | 13,75 | 10,96 | 22,35 |

Les compositions de teinture d'oxydation selon l'invention contiennent généralement environ 0,01 à 10% en poids, et de préférence environ 0,05 à 5% en poids, d'au moins une base d'oxydation de formule (I) ci-dessus définie.

L'agent oxydant est choisi de préférence parmi le peroxyde d'hydrogène, le peroxyde d'urée, les bromates de métaux alcalins, les persels tels que les perborates et les persulfates, et les enzymes telles que les peroxydases et les oxydo-réductases à deux électrons. L'utilisation du peroxyde d'hydrogène est particulièrement préférée.

La composition (A), qui renferme la base d'oxydation de formule (I) telle que décrite ci-dessus, peut avoir un pH compris entre 3 et 12, et de préférence compris entre 5 et 11, qui peut être ajusté à la valeur choisie au moyen soit d'agents alcalinisants habituellement utilisés en teinture des fibres kératiniques, tels que l'ammoniaque, les carbonates alcalins, les alcanolamines, par exemple les mono-, di- et triéthanolamines ainsi que leurs dérivés, les hydroxydes de sodium ou de potassium, les composés de formule :

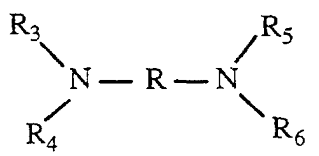

dans laquelle R est un reste propylène éventuellement substitué par un groupement hydroxyle ou un radical alkyle en $C_1$-$C_4$ ; $R_3$, $R_4$, $R_5$ et $R_6$, simultanément ou indépendamment l'un de l'autre, représentent un atome d'hydrogène, un radical alkyle en $C_1$-$C_4$ ou hydroxyalkyle en $C_1$-$C_4$,

soit au moyen d'agents acidifiants classiques, tels que les acides minéraux ou organiques comme par exemple les acides chlorhydrique, tartrique, citrique et phosphorique.

Le pH de la composition (B) renfermant l'agent oxydant tel que défini ci-dessus est tel qu'après un mélange avec la composition (A), le pH de la composition appliquée sur les fibres kératiniques humaines varie entre 3 et 12 et de préférence entre 5 et 11. Il est ajusté à la valeur désirée à l'aide d'agents acidifiants ou éventuellement alcalinisants bien connus de l'état de la technique, tels que ceux décrits ci-dessus.

La composition oxydante (B) est de préférence constituée par une solution d'eau oxygénée.

Selon un mode de réalisation préféré du procédé de teinture de l'invention, on mélange, au moment de l'emploi, la composition de teinture (A) décrite ci-dessus avec une solution oxydante en une quantité suffisante pour développer une coloration. Le mélange obtenu est ensuite appliqué sur les fibres kératiniques humaines et on laisse poser pendant 5 à 40 minutes, de préférence 15 à 30 minutes, après quoi on rince, on lave au shampooing, on rince à nouveau et on sèche.

Les compositions tinctoriales selon l'invention peuvent contenir, en plus des précurseurs de colorants d'oxydation de formule (I) définie ci-dessus, d'autres précurseurs de colorants d'oxydation choisis parmi les paraphénylènediamines, les bisphénylalkylènediamines, les para-aminophénols, les ortho-aminophénols, les bases hétérocycliques, les coupleurs benzéniques et les coupleurs hétérocycliques.

Parmi les paraphénylènediamines on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, et leurs sels d'addition avec un acide.

Parmi les bis-phénylalkylènediamines, on préfère le N,N'-bis(β-hydroxyéthyl) N,N'-bis(4'-aminophényl) 1,3-diaminopropanol ou l'un de ses sels d'addition avec un acide.

Parmi les para-aminophénols, on peut plus particulièrement citer le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut plus particulièrement citer le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut plus particulièrement citer les dérivés pyridiniques, les dérivés pyrimidiniques, les dérivés pyrazoliques, les dérivés pyrazolo-pyrimidiniques, et leurs sels d'addition avec un acide.

Parmi les coupleurs benzéniques, on peut plus particulièrement citer le méta-aminophénol, le 2-méthyl 5-aminophénol, le 2-méthyl-5-N-(β-hydroxyéthylamino)phénol, le 2-méthoxy-5-aminophénol, le 2-(β-hydroxyéthyloxy)-5-amino phénol, le 5-N-(β-hydroxyéthylamino) 4-méthoxy 2-méthyl phénol, le 5-amino 4-méthoxy 2-méthyl phénol, le 5-amino 4-chloro 2-méthyl phénol, le 5-amino 2,4-diméthoxy phénol, le 5-N-(γ-hydroxypropylamino) 2-méthyl phénol, le 3,5-diamino 1-éthyl 2-méthoxy benzène, le 3,5-diamino 2-méthoxy 1-méthyl benzène, le 2,4-diamino 1-éthoxy benzène, le 1,3bis(2,4-diaminophénoxy) propane, le bis-(2,4-diaminophénoxy) méthane, le 2,4-diamino phénoxy éthanol, le 1-(β-aminoéthyloxy) 2,4-diaminobenzène, le 2-amino 1-(β-hydroxyéthyloxy) 4-méthylamino benzène, le 1,3-diamino 4,6-bis-(β-hydroxyéthyloxy) benzène, le 2,4-diamino 1-éthoxy 5-méthyl benzène, le 2,4-diamino 5-(β-hydroxyéthyloxy) 1-méthyl benzène, le 2,4-diamino 1(β,γ-dihydroxypropyloxy) benzène, le 2-amino 4-N-(β-hydroxyéthylamino) anisole, le résorcinol et leurs sels d'addition avec un acide.

Parmi les coupleurs hétérocycliques, on peut citer les dérivés indoliques, les dérivés de benzomorpholine, les dérivés indoliniques, les dérivés de benzimidazole, les dérivés de sésamol, les dérivés pyrazolo-azoliques, les dérivés pyrrolo-azoliques, les dérivés imidazolo-azoliques, les dérivés pyrazolo-pyrimidiniques, les dérivés de pyrazolin-3,5-diones, les dérivés pyrrolo-[3,2-d]-oxazoliques, les dérivés pyrazolo-[3,4-d]-thiazoliques et les dérivés S-oxyde-thiazolo-azoliques et S,S-dioxyde-thiazolo-azoliques.

Les compositions tinctoriales selon l'invention peuvent aussi contenir des colorants directs choisis parmi les colorants azoïques, anthraquinoniques et les dérivés nitrés de la série benzénique et/ou des précurseurs de mélanine, notamment pour modifier les nuances ou les enrichir en reflets.

Les compositions de teinture peuvent également contenir des agents antioxydants. Ceux-ci peuvent être choisis en particulier parmi le sulfite de sodium, l'acide thioglycolique, l'acide thiolactique, le bisulfite de sodium, l'acide déhydroascorbique, l'hydroquinone, la 2-méthyl hydroquinone, la tert.butyl hydroquinone, la 3-méthyl 1-phényl 5-pyrazolone et l'acide homogentisique et sont alors généralement présents dans des proportions comprises entre environ 0,05 et 1,5% en poids par rapport au poids total de la composition.

Les compositions de teinture contiennent également, dans leur forme de réalisation préférée, des agents tensio-actifs bien connus de la technique, dans des proportions comprises entre environ 0,5 et 55% en poids, et de préférence entre 2 et 50% en poids par rapport au poids total de la composition, des solvants organiques, dans des proportions comprises entre environ 1 et 40% en poids, et en particulier entre 5 et 30% en poids par rapport au poids total de la composition, ou tout autre adjuvant cosmétiquement acceptable et connu de la technique antérieure en teinture d'oxydation capillaire.

La composition appliquée sur les cheveux peut se présenter sous des formes diverses, telles que sous forme de liquide, de crème, de gel, ou sous toute autre forme appropriée pour réaliser une teinture des fibres kératiniques, et notamment des cheveux humains. En particulier, elle peut être conditionnée sous pression en flacon aérosol en présence d'un agent propulseur et former une mousse.

Des exemples concrets illustrant l'invention vont maintenant être donnés.

## EXEMPLES DE TEINTURE

### EXEMPLES 1 à 10 DE TEINTURE EN MILIEU NEUTRE

On prépare les compositions tinctoriales suivantes (teneurs en grammes):

| EXEMPLE | 1 | 2 | 3 | 4 | 5 | 6 | 7 | 8 | 9 | 10 |
|---|---|---|---|---|---|---|---|---|---|---|
| Trichlorhydrate de Nε-(4-aminophényl)-L-lysine (Base d'oxydation conforme à l'invention) | 1,038 | 1,038 | 1,038 | 1,038 | 1,038 | - | - | - | - | - |
| Dichlorhydrate de N-(4-aminophényl)-glycine (Base d'oxydation conforme à l'invention) | - | - | - | - | - | 0,717 | 0,717 | 0,717 | 0,717 | 0,717 |
| Para-aminophénol (Base d'oxydation additionnelle) | - | - | 0,327 | - | - | - | - | 0,327 | - | - |
| 2-méthyl 5-N-(β-hydroxyéthyl) amino phénol (Coupleur) | - | 0,543 | - | - | - | - | 0,543 | - | - | - |
| Résorcine (Coupleur) | - | - | - | 0,33 | - | - | - | - | 0,33 | - |
| Méta-aminophénol (Coupleur) | - | - | - | - | 0,327 | - | - | - | - | 0,327 |
| Support de teinture commun | (*) | (*) | (*) | (*) | (*) | (*) | (*) | (*) | (*) | (*) |
| Eau déminéralisée q. s.p. | 100 g | 100 g | 100 g | 100 g | 100 g | 100 g | 100 g | 100 g | 100 g | 100 g |

Les colorants sont utilisés en quantité molaire égale, à savoir 3 x $10^{-3}$ mole %.
(*) Support de teinture commun :
- Ethanol à 96° 18 g
- Sel pentasodique de l'acide diéthylène triamine pentacétique vendu sous la dénomination MASQUOL DTPA par la société PROTEX 1,1 g
- Métabisulfite de sodium en solution aqueuse à 35 % de M.A. 0,68 g M.A.
- $K_2 HPO_4/KH_2 PO_4$ (1,5 M/1 M) 10 g

Au moment de l'emploi, on mélange poids pour poids chacune des compositions tinctoriales ci-dessus avec une solution de peroxyde d'hydrogène à 20 volumes (6 % en poids).

Le mélange obtenu est appliqué sur des mèches de cheveux gris naturels à 90 % de blancs à raison de 30 g pour 3 g de cheveux pendant 30 minutes. Les mèches sont ensuite rincées, lavées avec un shampooing standard, rincées à nouveau puis séchées.

La coloration capillaire est évaluée de manière visuelle puis mesurée au colorimètre MINOLTA CM 2002.

Les nuances obtenues figurent dans le tableau ci-après :

| EXEMPLE | Ton | Nuance sur cheveux naturels |
|---|---|---|
| 1 | 6 | Blond foncé cendré mat |
| 2 | 6 | Blond foncé violine cendré |

(suite)

| EXEMPLE | Ton | Nuance sur cheveux naturels |
|---|---|---|
| 3 | 6 | Blond foncé naturel doré |
| 4 | 6 | Blond foncé gris |
| 5 | 6 | Blond foncé vert |
| 6 | 6 | Blond foncé gris légèrement violacé |
| 7 | 6 | Blond foncé violine irisé |
| 8 | 5 | Châtain clair naturel cendré |
| 9 | 7,5 | Blond clair beige doré |
| 10 | 5 | Châtain clair vert gris |

**EXEMPLES 11 à 20 DE TEINTURE EN MILIEU BASIQUE**

On prépare les compositions tinctoriales suivantes (teneurs en grammes) :

| EXEMPLE | 11 | 12 | 13 | 14 | 15 | 16 | 17 | 18 | 19 | 20 |
|---|---|---|---|---|---|---|---|---|---|---|
| Trichlorhydrate de Nε-(4-aminophényl)-L-lysine (Base d'oxydation conforme à l'invention) | 1,038 | 1,038 | 1,038 | 1,038 | 1,038 | - | - | - | - | - |
| Dichlorhydrate de N-(4-aminophényl)-glycine (Base d'oxydation conforme à l'invention) | - | - | - | - | - | 0,717 | 0,717 | 0,717 | 0,717 | 0,717 |
| Para-aminophénol (Base d'oxydation additionnelle) | - | - | 0,327 | - | - | - | - | 0,327 | - | - |
| 2-méthyl 5-N-(β-hydroxyéthyl) amino phénol (Coupleur) | - | 0,543 | - | - | - | - | 0,543 | - | - | - |
| Résorcine (Coupleur) | - | - | - | 0,33 | - | - | - | - | 0,33 | - |
| Méta-aminophénol (Coupleur) | - | - | - | - | 0,327 | - | - | - | - | 0,327 |

(suite)

| EXEMPLE | 11 | 12 | 13 | 14 | 15 | 16 | 17 | 18 | 19 | 20 |
|---|---|---|---|---|---|---|---|---|---|---|
| Support de teinture commun | (*) | (*) | (*) | (*) | (*) | (*) | (*) | (*) | (*) | (*) |
| Eau déminéralisée q. s.p. | 100 g | 100 g | 100 g | 100 g | 100 g | 100 g | 100 g | 100 g | 100 g | 100 g |

Les colorants sont utilisés en quantité molaire égale, à savoir 3 x $10^{-3}$ mole %.
(*) Support de teinture commun :
- Ethanol à 96° 18 g
- Sel pentasodique de l'acide diéthylène triamine pentacétique vendu sous la dénomination MASQUOL DTPA par la société PROTEX 1,1 g
- Métabisulfite de sodium en solution aqueuse à 35 % de M.A. 0,68 g M.A.
- Ammoniaque à 20% 10 g

Le mode d'application des teintures est identique à celui utilisé dans les exemples 1 à 10.
La coloration capillaire est évaluée de la même manière.
Les nuances obtenues figurent dans le tableau ci-après :

| EXEMPLE | Ton | Nuance sur cheveux naturels |
|---|---|---|
| 11 | 8 | Blond clair vert gris |
| 12 | 7 | Blond violine |
| 13 | 7,5 | Blond doré mat |
| 14 | 7,5 | Blond clair gris beige |
| 15 | 6,5 | Blond gris bleuté |
| 16 | 8,5 | Blond clair à reflet vert jaune |
| 17 | 8 | Blond clair violine |
| 18 | 6,5 | Blond doré mat |
| 19 | 8,5 | Blond clair à reflet vert jaune |
| 20 | 7,5 | Blond à reflet gris |

Les hauteurs de "ton" mentionnées dans les tableaux ci-dessus reposent sur la classification des nuances naturelles ci-après, un ton séparant chaque nuance de celle qui la suit ou la précède immédiatement (voir "Science des Traitements Capillaires" de C. ZVIAK, Ed. Masson 1988, p. 278).
Cette classification est la suivante :

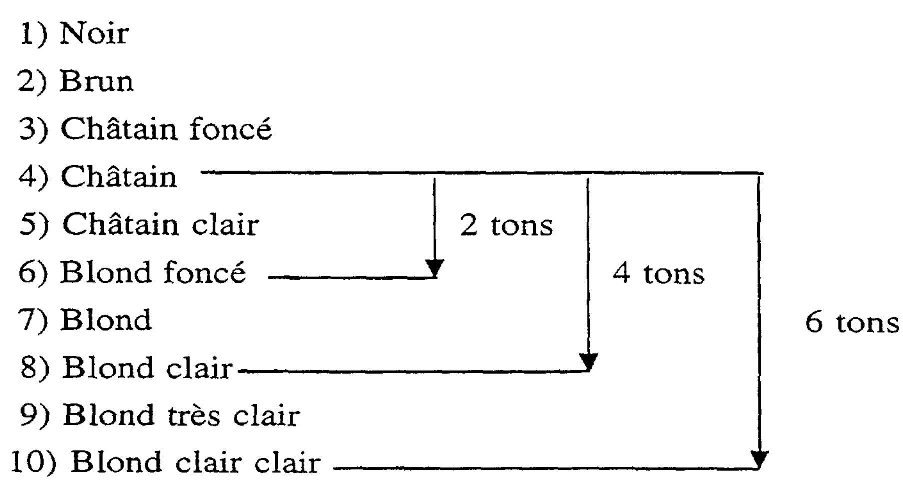

En comparant les exemples 1 à 5 réalisés en milieu neutre aux exemples 11 à 15 réalisés en milieu basique avec la même base d'oxydation, à savoir la Nε-(4-aminophényl)-L-lysine, on s'aperçoit que cette dernière est plus puissante en milieu neutre qu'en milieu basique, car les hauteurs de ton obtenues en milieu basique sont plus élevées de 0,5 à 2 tons, c'est-à-dire plus claires que celles obtenues en milieu neutre avec les mêmes précurseurs de colorants.

De même, si l'on compare les exemples 6 à 10 aux exemples 16 à 20 dans lesquels on utilise la N-(4-aminophényl)-glycine, on observe que les teintures obtenues en milieu basique ont des hauteurs de ton plus élevées de 1 à 2,5 tons, c'est-à-dire sont plus claires que celles obtenues en milieu neutre avec les mêmes précurseurs de colorants d'oxydation.

## Revendications

1. Dérivés d'aminoacides substitués sur un groupe amino par un groupe à noyau aromatique de formule :

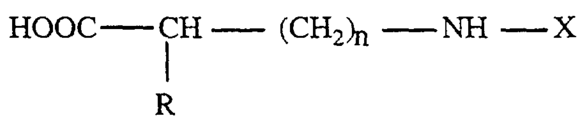

(I')

dans laquelle :

- n est un nombre entier compris entre 0 et 11 inclusivement ;
- R représente un atome d'hydrogène, un radical amino, alkyle en $C_1$-$C_6$ linéaire ou ramifié, saturé ou insaturé, ou un radical choisi parmi les groupements suivants :

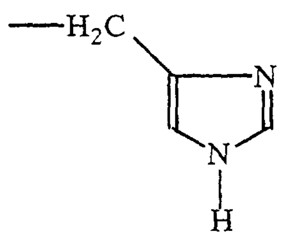

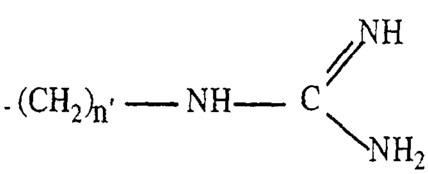

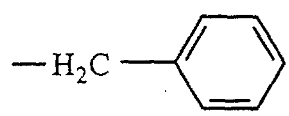

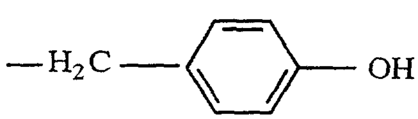

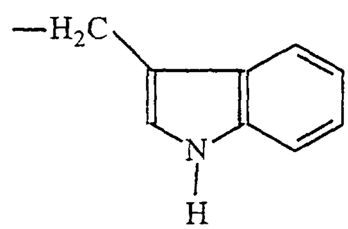

$-(CH_2)_{n'}-COOH$ 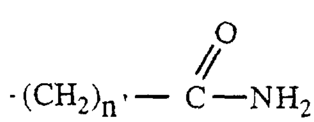 $-(CH_2)_{n'}-SH$

$-(CH_2)_{n'}-SR'$ $-CH_2OH$ $-CHOH-R'$

$-(CH_2)_{n'}—NH—X$ et $-(CH_2)_{n'}—NH_2$

- R' représente un radical alkyle en $C_1$-$C_6$, linéaire ou ramifié, saturé ou insaturé ;
- n' est un nombre entier compris entre 1 et 6 inclusivement ;
- X représente un groupe aromatique à un ou deux cycles, le ou les cycles pouvant contenir un ou plusieurs hétéroatomes choisis parmi S, N ou O ; le ou les cycles étant substitués par au moins un groupement -NHR" ou par un radical -OH se trouvant en position para ou ortho sur le groupe aromatique X par rapport au groupement -NH, R" désignant un atome d'hydrogène, un radical alkyle en $C_1$-$C_6$, linéaire ou ramifié, saturé ou insaturé, éventuellement hydroxylé, sans comporter de groupements nitro,

à l'exclusion des composés dans lesquels n=0, R=H et X est un radical 4-aminophényle, 4-hydroxyphényle, 3-hydroxy-2-pyridyle ou 4-hydroxy-3-pyridyle, du composé dans lequel n=0, R=-$CH_3$ et X est un radical 3-hydroxy-2-pyridyle et du composé dans lequel n=0, R = -$CH_2OH$ et X est un radical 3,4-dihydroxy-6-méthylphényle,

ou l'un de leurs sels d'addition avec un acide.

2. Composé selon la revendication 1, **caractérisé par le fait que** X représente un cycle phényle ou pyridyle substitué par un groupement amino -$NH_2$.

3. Composé selon la revendication 1 ou 2, **caractérisé par le fait qu'**il est choisi parmi : la Nε-(4-aminophényl)-L-lysine ; la Nε-(4-amino-2-pyridyl)-L-lysine; la Nα-(4-aminophényl)-L-lysine; la Nα,Nε-di-(4-aminophényl)-L-lysine ; la N-(4-aminophényl)-L-sérine, l'acide 6-N-(4-aminophényl)amino-caproïque et l'α-N-(4-aminophényl)amino -ε-caprolactame, ainsi que leurs sels d'addition avec un acide.

4. Composé selon l'une quelconque des revendications 1 à 3 **caractérisé par le fait que** les sels d'addition avec un acide sont choisis parmi les chlorhydrates, les sulfates, les bromhydrates et les tartrates.

5. Composition de teinture d'oxydation pour fibres kératiniques, en particulier pour fibres kératiniques humaines telles que les cheveux, **caractérisée par le fait qu'**elle comprend, dans un milieu approprié pour la teinture, à titre de base d'oxydation, au moins un dérivé d'aminoacide substitué sur un groupe amino par un groupe à noyau aromatique de formule :

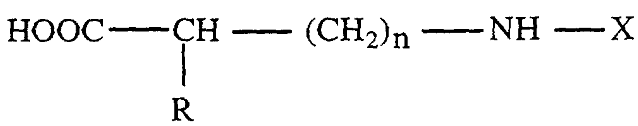

(I)

dans laquelle :

- n est un nombre entier compris entre 0 et 11 inclusivement ;
- R représente un atome d'hydrogène, un radical amino, alkyle en $C_1$-$C_6$ linéaire ou ramifié, saturé ou insaturé, ou un radical choisi parmi les groupements suivants :

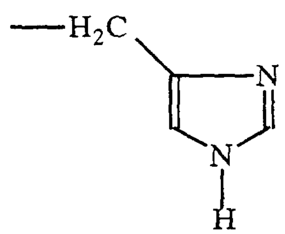

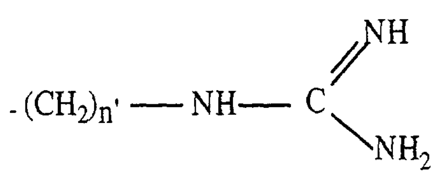

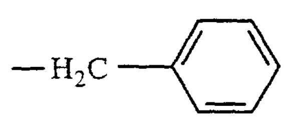

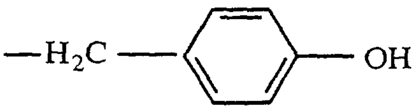

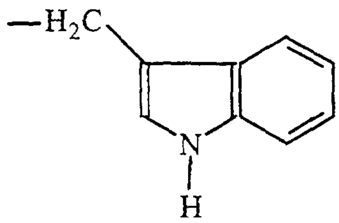

$-(CH_2)_{n'}-COOH$

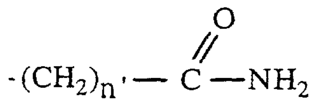

$-(CH_2)_{n'}-SH$

$-(CH_2)_{n'}-SR'$ $-CH_2OH$ $-CHOH-R'$

$-(CH_2)_{n'}-NH-X$ et $-(CH_2)_{n'}-NH_2$

- R' représente un radical alkyle en $C_1$-$C_6$, linéaire ou ramifié, saturé ou insaturé ;
- n' est un nombre entier compris entre 1 et 6 inclusivement ;
- X représente un groupe aromatique à un ou deux cycles, le ou les cycles pouvant contenir un ou plusieurs hétéroatomes choisis parmi S, N ou O ; le ou les cycles étant substitués par au moins un groupement -NHR" ou par un radical -OH se trouvant en position para ou ortho sur le groupe aromatique X par rapport au groupement -NH, R" désignant un atome d'hydrogène, un radical alkyle en $C_1$-$C_6$, linéaire ou ramifié, saturé ou insaturé, éventuellement hydroxylé, sans comporter de groupements nitro, à l'exclusion du composé dans lequel n=0, R=H et X est un radical 4-hydroxyphényle,

ou l'un de ses sels d'addition avec un acide.

**6.** Composition tinctoriale selon la revendication 5, **caractérisée par le fait qu'**elle contient au moins un dérivé d'aminoacide substitué sur un groupe amino par un groupe à noyau aromatique de formule (I), dans laquelle X est un radical phényle ou pyridyle substitué par un groupe amino $-NH_2$.

**7.** Composition tinctoriale selon la revendication 5 ou 6 **caractérisée par le fait qu'**elle contient au moins un dérivé d'aminoacide choisi parmi la Nε-(4-aminophényl)-L-lysine, la Nε-(4-amino-2-pyridyl)-L-lysine, la N-(4-aminophényl)-glycine, la Nα-(4-aminophényl)-L-lysine, la Nα,Nε-di(4-aminophényl)-L-lysine, la N-(4-aminophényl)-L-sérine, l'acide 6-N-(4-aminophényl)amino-caproïque et l'α-N-(4-aminophényl)amino -ε-caprolactame ainsi que leurs sels d'addition avec un acide.

**8.** Composition tinctoriale selon l'une quelconque des revendications 5 à 7, **caractérisée par le fait que** les sels d'addition avec un acide sont choisis parmi les chlorhydrates, les sulfates, les bromhydrates et les tartrates.

**9.** Composition tinctoriale selon l'une quelconque des revendications 5 à 8, **caractérisée par le fait qu'**elle contient 0,01 à 10% en poids, et de préférence 0,05 à 5% en poids d'au moins une base d'oxydation de formule (I).

**10.** Composition tinctoriale selon l'une quelconque des revendications 5 à 9, prête à l'emploi, **caractérisée en ce qu'**elle contient en outre un agent oxydant et qu'elle possède un pH compris entre 3 et 12, et de préférence entre 5 et 11.

**11.** Composition tinctoriale selon l'une quelconque des revendications 5 à 10, **caractérisée par le fait qu'**elle contient d'autres précurseurs de colorants d'oxydation choisis parmi les paraphénylènediamines, les bis-phénylalkylènediamines, les para-aminophénols, les ortho-aminophénols, les bases hétérocycliques, les coupleurs benzéniques et les coupleurs hétérocycliques.

**12.** Composition tinctoriale selon l'une quelconque des revendications 5 à 11, **caractérisée par le fait qu'**elle contient en outre des colorants directs choisis parmi les colorants azoïques, anthraquinoniques et les dérivés nitrés de la série benzénique ou des précurseurs de mélanine.

**13.** Procédé de teinture des fibres kératiniques et en particulier des fibres kératiniques humaines telles que les cheveux, **caractérisé par le fait qu'**il consiste à appliquer sur les fibres une composition tinctoriale (A) selon l'une quelconque des revendications 5 à 12, et à révéler la couleur en milieu acide, neutre ou alcalin à l'aide d'un agent oxydant ajouté juste au moment de l'emploi à cette composition (A) ou présent dans une composition (B), appliquée simultanément ou séquentiellement de façon séparée.

**14.** Dispositif à plusieurs compartiments ou "kit" pour la teinture des fibres kératiniques et en particulier des fibres kératiniques humaines telles que les cheveux, **caractérisé par le fait qu'**il comporte au moins deux compartiments, dont l'un d'entre eux renferme une composition (A) telle que définie dans l'une quelconque des revendications 5 à 12, et un autre renferme une composition (B) comprenant un agent oxydant dans un milieu approprié pour la teinture.

**Claims**

**1.** Amino acid derivatives substituted on an amino group by a group with an aromatic nucleus of formula:

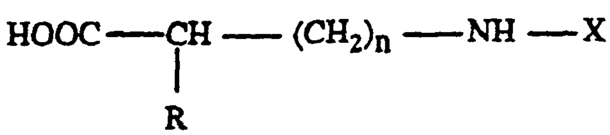
(I')

in which:

- n is an integer between 0 and 11 inclusive;
- R represents a hydrogen atom, an amino radical, a saturated or unsaturated, linear or branched, $C_1$-$C_6$ alkyl radical or a radical chosen from the following groups:

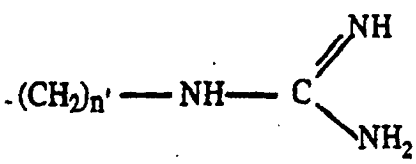
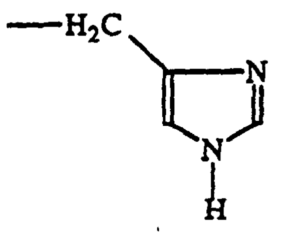
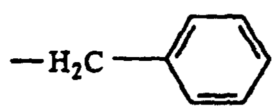
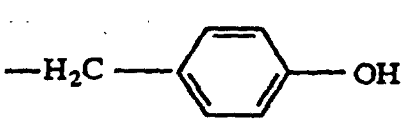
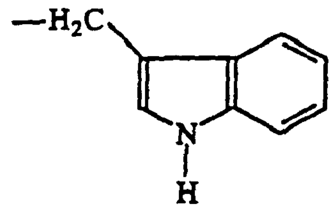

$-(CH_2)_{n'}—COOH$ 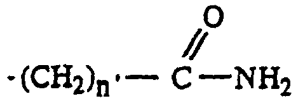 $-(CH_2)_{n'}—SH$

$-(CH_2)_{n'}—SR'$ $-CH_2OH$ $-CHOH—R'$

$-(CH_2)_{n'}—NH—X$ and $-(CH_2)_{n'}—NH_2$

- R' represents a saturated or unsaturated, linear or branched, $C_1$-$C_6$ alkyl radical;
- n' is an integer between 1 and 6 inclusive;
- X represents an aromatic group with one or two rings, it being possible for the ring or rings to comprise one or more heteroatoms chosen from S, N or O; the ring or rings being substituted by at least one -NHR" group or by an -OH radical which is in the para or ortho position on the aromatic group X with respect to the -NH group, R" denoting a hydrogen atom or an optionally hydroxylated, saturated or unsaturated, linear or branched, $C_1$-$C_6$ alkyl radical, without comprising nitro groups,

with the exception of the compounds in which n = 0, R = H and X is a 4-aminophenyl, 4-hydroxyphenyl, 3-hydroxy-2-pyridyl or 4-hydroxy-3-pyridyl radical, of the compound in which n = 0, R = $-CH_3$ and X is a 3-hydroxy-2-pyridyl radical and of the compound in which n = 0, R = $-CH_2OH$ and X is a 3,4-dihydroxy-6-methylphenyl radical, or one of their addition salts with an acid.

**2.** Compound according to Claim 1, **characterized in that** X represents a phenyl or pyridyl ring substituted by an amino group $-NH_2$.

**3.** Compound according to Claim 1 or 2, **characterized in that** it is chosen from: Nε-(4-aminophenyl)-L-lysine, Nε-(4-amino-2-pyridyl)-L-lysine, Nα-(4-aminophenyl)-L-lysine, Nα,Nε-di(4-aminophenyl)-L-lysine, N-(4-aminophenyl)-L-serine, 6-[N-(4-aminophenyl)amino]caproic acid and α-N-(4-aminophenyl)amino-ε-caprolactam, and their addition salts with an acid.

**4.** Compound according to any one of Claims 1 to 3, **characterized in that** the addition salts with an acid are chosen from hydrochlorides, sulphates, hydrobromides and tartrates.

**5.** Oxidation dyeing composition for keratinous fibres, in particular for human keratinous fibres, such as hair **characterized in that** it comprises, in a medium appropriate for dyeing, as oxidation base, at least one amino acid derivative substituted on an amino group by a group with an aromatic nucleus of formula:

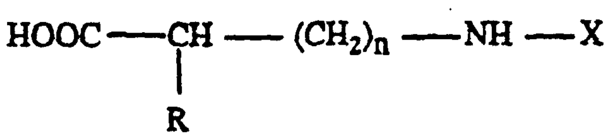 (I)

in which:

- n is an integer between 0 and 11 inclusive;
- R represents a hydrogen atom, an amino radical, a saturated or unsaturated, linear or branched, $C_1$-$C_6$ alkyl radical or a radical chosen from the following groups:

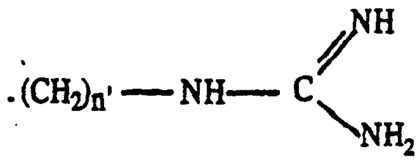

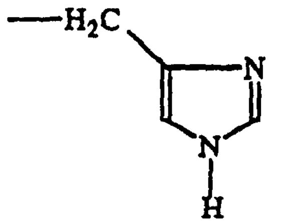

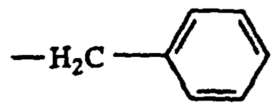

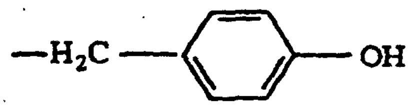

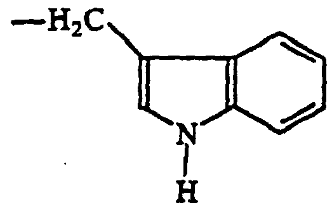

$-(CH_2)_{n'}$— COOH

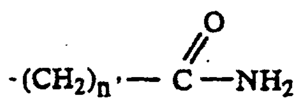

$-(CH_2)_{n'}$—SH

$-(CH_2)_{n'}$— SR' $-CH_2OH$ -CHOH— R'

$-(CH_2)_{n'}$— NH— X and $-(CH_2)_{n'}$— $NH_2$

- R' represents a saturated or unsaturated, linear or branched, $C_1$-$C_6$ alkyl radical;
- n' is an integer between 1 and 6 inclusive;
- X represents an aromatic group with one or two rings, it being possible for the ring or rings to comprise one or more heteroatoms chosen from S, N or O; the ring or rings being substituted by at least one -NHR" group or by an -OH radical which is in the para or ortho position on the aromatic group X with respect to the -NH group, R" denoting a hydrogen atom or an optionally hydroxylated, saturated or unsaturated, linear or branched, $C_1$-$C_6$ alkyl radical, without comprising nitro groups, with the exception of the compound in which n = 0, R = H and X is a 4-hydroxyphenyl radical,

or one of its addition salts with an acid.

6. Dyeing composition according to Claim 5, **characterized in that** it comprises at least one amino acid derivative substituted on an amino group by a group with an aromatic nucleus of formula (I), in which X is a phenyl or pyridyl radical substituted by an amino group $-NH_2$.

7. Dyeing composition according to Claim 5 or 6, **characterized in that** it comprises at least one amino acid derivative chosen from Nε-(4-aminophenyl)-L-lysine, Nε-(4-amino-2-pyridyl)-L-lysine, N-(4-aminophenyl)-glycine, Nα-(4-aminophenyl)-L-lysine, Nα,Nε-di(4-aminophenyl)-L-lysine, N-(4-aminophenyl)-L-serine, 6-[N-(4-aminophenyl) amino]caproic acid and α-N-(4-aminophenyl)amino-ε-caprolactam, and their addition salts with an acid.

8. Dyeing composition according to any one of Claims 5 to 7, **characterized in that** the addition salts with an acid are chosen from hydrochlorides, sulphates, hydrobromides and tartrates.

9. Dyeing composition according to any one of Claims 5 to 8, **characterized in that** it comprises 0.01 to 10% by weight, preferably 0.05 to 5% by weight, of at least one oxidation base of formula (I).

**10.** Ready-for-use dyeing composition according to any one of Claims 5 to 9, **characterized in that** it additionally comprises an oxidizing agent and **in that** it has a pH of between 3 and 12, preferably between 5 and 11.

**11.** Dyeing composition according to any one of Claims 5 to 10, **characterized in that** it comprises other oxidation dye precursors chosen from para-phenylenediamines, bisphenylalkylenediamines, para-aminophenols, ortho-aminophenols, heterocyclic bases, benzene couplers and heterocyclic couplers.

**12.** Dyeing composition according to any one of Claims 5 to 11, **characterized in that** it additionally comprises direct dyes chosen from azo or anthraquinone dyes and nitro derivatives of the benzene series or melanin precursors.

**13.** Process for dyeing keratinous fibres and in particular human keratinous fibres, such as hair, **characterized in that** it consists in applying, to the fibres, a dyeing composition (A) according to any one of Claims 5 to 12 and in developing the colour in acidic, neutral or alkaline medium using an oxidizing agent added only at the time of use to this composition (A) or present in a composition (B) applied simultaneously or sequentially in a separate fashion.

**14.** Multi-compartment device or "kit" for dyeing keratinous fibres and in particular human keratinous fibres, such as hair, **characterized in that** it comprises at least two compartments, one of which includes a composition (A) as defined in any one of Claims 5 to 12 and another of which includes a composition (B) comprising an oxidizing agent in a medium appropriate for dyeing.

**Patentansprüche**

**1.** Aminosäurederivate, die an einer Aminogruppe mit einer Gruppe mit aromatischem Ring substituiert sind, der folgenden Formel:

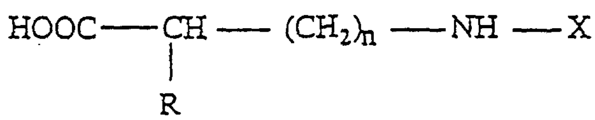

(I')

worin bedeuten:

- n 0 oder eine ganze Zahl von 1 bis 11;
- R ein Wasserstoffatom, eine Aminogruppe, eine geradkettige oder verzweigte, gesättigte oder ungesättigte $C_{1-6}$-Alkylgruppe oder eine Gruppe, die unter den folgenden Gruppen ausgewählt ist:

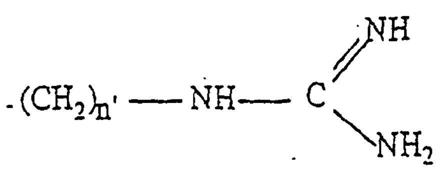

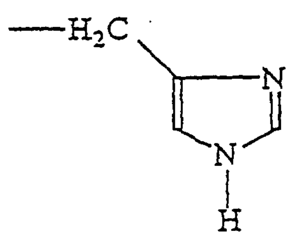

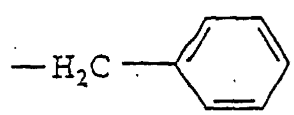

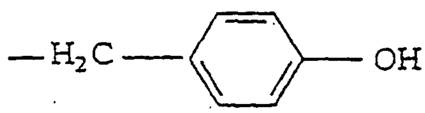

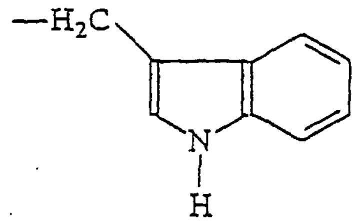

$-(CH_2)_{n'}-COOH$

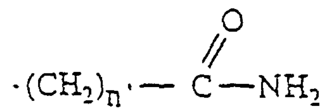

$-(CH_2)_{n'}-SH$

$-(CH_2)_{n'}-SR'$ $-CH_2OH$ $-CHOH-R'$

$-(CH_2)_{n'}-NH-X$ und $-(CH_2)_{n'}-NH_2$

- R' eine geradkettige oder verzweigte, gesättigte oder ungesättigte $C_{1-6}$-Alkylgruppe;
- n' eine ganze Zahl von 1 bis 6;
- X eine aromatische Gruppe mit einem oder zwei Ringen, wobei der Ring oder die Ringe ein oder mehrere Heteroatome enthalten können, die unter S, N oder O ausgewählt sind; wobei der Ring oder die Ringe mit mindestens einer Gruppe -NHR" oder einer Gruppe -OH substituiert sind, die sich in bezug auf die Gruppe -NH an der aromatischen Gruppe X in p- oder o-Stellung befinden, wobei R" ein Wasserstoffatom oder eine geradkettige oder verzweigte, gesättigte oder ungesättigte, gegebenenfalls hydroxylierte $C_{1-6}$-Alkylgruppe bedeutet, wobei keine Nitrogruppen enthalten sind, mit Ausnahme von Verbindungen, worin bedeuten: n = 0, R = H und X = 4-Aminophenyl, 4-Hydroxyphenyl, 3-Hydroxy-2-pyridyl oder 4-Hydroxy-3-pyridyl, der Verbindung, worin bedeuten: n = 0, R = $-CH_3$ und X = 3-Hydroxy-2-pyridyl, und der Verbindung, worin bedeuten: n = 0, R = $-CH_2OH$ und X = 3,4-Dihydroxy-6-methylphenyl,

oder
die Additionssalze dieser Verbindungen mit einer Säure.

2. Verbindung nach Anspruch 1, **dadurch gekennzeichnet, daß** X einen Phenyl- oder Pyridylring bedeutet, der mit einer Aminogruppe $-NH_2$ substituiert ist.

3. Verbindung nach Anspruch 1 oder 2, **dadurch gekennzeichnet, daß** sie ausgewählt ist unter: Nε-(4-Aminophenyl)-L-lysin; Nε-(4-Amino-2-pyridyl)-L-lysin; Nα-(4-Aminophenyl)-L-lysin; Nα,Nε-Di-(4-aminophenyl)-L-lysin; N-(4-Aminophenyl)-L-serin, 6-N-(4-Aminophenyl)amino-capronsäure und α-N-(4-Aminophenyl)amino-ε-caprolactam sowie deren Additionssalzen mit einer Säure.

4. Verbindung nach einem der Ansprüche 1 bis 3, **dadurch gekennzeichnet, daß** die Additionssalze mit einer Säure unter den Hydrochloriden, Sulfaten, Hydrobomiden und Tartraten ausgewählt sind.

5. Zusammensetzung zum oxidativen Färben von Keratinfasern und insbesondere menschlichen Keratinfasern wie dem Haar, **dadurch gekennzeichnet, daß** sie in einem zum Färben geeigneten Medium als Oxidationsbase mindestens eine Aminosäure, die an einer Aminogruppe mit einer Gruppe mit aromatischem Ring substituiert ist, der folgenden Formel enthält:

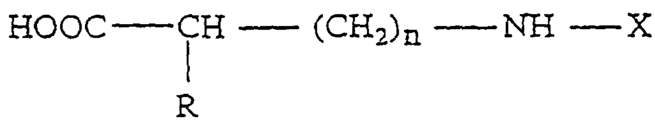

(I)

worin bedeuten:

- n 0 oder eine ganze Zahl von 1 bis 11;
- R ein Wasserstoffatom, eine Aminogruppe, eine geradkettige oder verzweigte, gesättigte oder ungesättigte $C_{1-6}$-Alkylgruppe oder eine Gruppe, die unter den folgenden Gruppen ausgewählt ist:

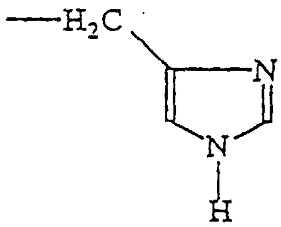

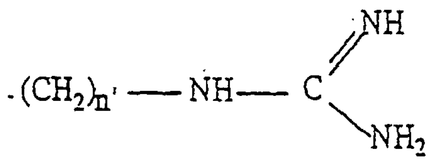

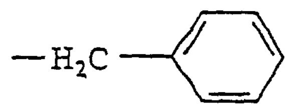

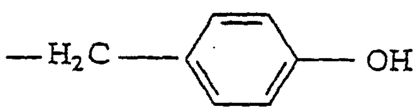

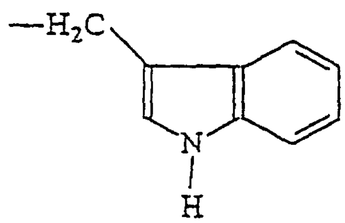

$-(CH_2)_{n'}-COOH$ 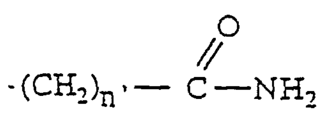 $-(CH_2)_{n'}-SH$

$-(CH_2)_{n'}-SR'$ $-CH_2OH$ $-CHOH-R'$

$-(CH_2)_{n'}-NH-X$ und $-(CH_2)_{n'}-NH_2$

- R' eine geradkettige oder verzweigte, gesättigte oder ungesättigte $C_{1-6}$-Alkylgruppe;
- n' eine ganze Zahl von 1 bis 6;
- X eine aromatische Gruppe mit einem oder zwei Ringen, wobei der Ring oder die Ringe ein oder mehrere Heteroatome enthalten können, die unter S, N oder O ausgewählt sind;
  wobei der Ring oder die Ringe mit mindestens einer Gruppe -NHR" oder einer Gruppe -OH substituiert sind, die sich in bezug auf die Gruppe -NH an der aromatischen Gruppe X in p- oder o-Stellung befinden, wobei R" ein Wasserstoffatom oder eine geradkettige oder verzweigte, gesättigte oder ungesättigte, gegebenenfalls hydroxylierte $C_{1-6}$-Alkylgruppe bedeutet, wobei keine Nitrogruppen enthalten sind,
  wobei die Verbindung ausgeschlossen ist, worin bedeuten: n = O, R = H und X = 4-Hydroxyphenyl;

oder

eines der Additionssalze dieser Verbindungen mit einer Säure.

6. Zusammensetzung zum Färben nach Anspruch 5, **dadurch gekennzeichnet, daß** sie mindestens ein Aminosäurederivat, das an einer Aminogruppe mit einer Gruppe mit aromatischem Ring substituiert ist, der Formel (I) enthält, worin X eine Phenyl- oder Pyridylgruppe bedeutet, die mit einer Aminogruppe $-NH_2$ substituiert ist.

7. Zusammensetzung zum Färben nach Anspruch 5 oder 6, **dadurch gekennzeichnet, daß** sie mindestens eine Aminosäure enthält, die ausgewählt ist unter: Nε-(4-Aminophenyl)-L-lysin; Nε-(4-Amino-2-pyridyl)-L-lysin; N-(4-Aminophenyl)-L-glycin; Nα-(4-Aminophenyl)-L-lysin; Nα,Nε-Di-(4-aminophenyl)-L-lysin; N-(4-Aminophenyl)-L-serin, 6-N-(4-Aminophenyl)amino-capronsäure und α-N-(4-Aminophenyl)amino-ε-caprolactam sowie deren Additionssalzen mit einer Säure.

8. Zusammensetzung zum Färben nach einem der Ansprüche 5 bis 7, **dadurch gekennzeichnet, daß** die Additionssalze mit einer Säure unter den Hydrochloriden, Sulfaten, Hydrobromiden und Tartraten ausgewählt sind.

9. Zusammensetzung zum Färben nach einem der Ansprüche 5 bis 8, **dadurch gekennzeichnet, daß** sie 0,01 bis 10 Gew.-% und vorzugsweise 0,05 bis 5 Gew.-% mindestens einer Oxidationsbase der Formel (I) enthält.

10. Zusammensetzung zum Färben nach einem der Ansprüche 5 bis 9, die gebrauchsfertig ist, **dadurch gekennzeichnet, daß** sie ferner ein Oxidationsmittel enthält und einen pH-Wert im Bereich von 3 bis 12 und vorzugsweise 5 bis 11 aufweist.

11. Zusammensetzung zum Färben nach einem der Ansprüche 5 bis 10, **dadurch gekennzeichnet, daß** sie weitere Farbstoffvorprodukte von Oxidationsfarbstoffen enthält, die unter den p-Phenylendiaminen, Bisphenylalkylendiaminen, p-Aminophenolen, o-Aminophenolen, heterocyclischen Basen, Benzolkupplern und heterocyclischen Kupplern ausgewählt sind.

12. Zusammensetzung zum Färben nach einem der Ansprüche 5 bis 11, **dadurch gekennzeichnet, daß** sie ferner Direktfarbstoffe enthält, die unter den Azofarbstoffen, Anthrachinonfarbstoffen, nitrierten Derivaten aus der Benzolgruppe oder Melaninprecursoren ausgewählt sind.

13. Verfahren zum Färben von Keratinfasern und insbesondere menschlichen Keratinfasern, wie dem Haar, **dadurch gekennzeichnet, daß** es darin besteht, auf die Fasern eine Farbmittelzusammensetzung (A) nach einem der Ansprüche 5 bis 12 aufzutragen und die Farbe bei einem sauren, neutralen oder alkalischen pH-Wert mit einem Oxidationsmittel zu entwickeln, das bei der Anwendung zu der Zusammensetzung (A) gegeben wird oder in einer Zusammensetzung (B) vorliegt, die gleichzeitig oder getrennt davon anschließend aufgetragen wird.

14. Vorrichtung mit mehreren Abteilungen oder "Kit" zum Färben von Keratinfasern und insbesondere menschlichen Keratinfasern wie dem Haar, **dadurch gekennzeichnet, daß** sie mindestens zwei Abteilungen aufweist, wobei eine Abteilung eine Zusammensetzung (A) nach einem der Ansprüche 5 bis 12 und eine andere Abteilung eine Zusammensetzung (B) enthält, die in einem zum Färben geeigneten Medium ein Oxidationsmittel enthält.